# EUROPEAN PATENT SPECIFICATION

(11) **EP 1 523 542 B1**
(45) Date of publication and mention of the grant of the patent: **23.05.2007**
(21) Application number: 03735234.1
(22) Date of filing: 11.07.2003
(51) Int. Cl.: C11B 9/00, C07D 309/18

(54) **FRAGRANCE COMPOSITION**
DUFTSTOFFZUSAMMENSETZUNG
COMPOSITION AROMATIQUE

(30) Priority: 20.07.2002 GB 0216940
(43) Date of publication of application: 20.04.2005
(73) Proprietor: Givaudan SA, 1214 Vernier-Genève (CH)
(72) Inventor: KRAFT, Philip, CH-8600 Dübendorf (CH); CADALBERT, Riccardo, CH-8003 Zürich (CH)
(74) Representative: McStea, John Anthony
(86) International application number: PCT/CH2003/000466
(87) International publication number: WO 2004/009749

(56) References cited:
- US-A- 3 681 263
- DATABASE WPI Derwent Publications Ltd., London, GB; AN 1982-11549E XP002256431 & SU 825 528 A
- DATABASE WPI Derwent Publications Ltd., London, GB; AN 1986-054437 XP002256432 & SU 1 171 459 A
- HANAMOTO, TAKESHI ET AL: "Scandium(III) perfluorooctanesulfonate [Sc(OPf)3]: a novel catalyst for the hetero Diels-Alder reaction of aldehydes with non-activated dienes" BULLETIN OF THE CHEMICAL SOCIETY OF JAPAN (1997), 70(6), 1421-1426 , XP009018459
- OI, SHUICHI ET AL: "Cationic palladium(II) complex-catalyzed hetero Diels-Alder reaction of dienes with aldehydes" TETRAHEDRON LETTERS (1996), 37(35), 6351-6354 , XP004030687
- ROMANOV, N. A. ET AL: "Transcyclization of 4,4,5-trimethyl-1,3-dioxane by carbonyl compounds" J.ORG.CHEM.USSR, vol. 18, no. 2, 1982, pages 399-400, XP009018460

## Description

The invention relates to new fragrance ingredients, their manufacture and use in fragrance compositions, particularly in fine perfumery and cosmetics compositions.

During their investigations of the low-boiling constituents of Bulgarian rose oil in 1959, C. F. Seidel and M. Stoll (*Helv. Chim. Acta* **1959**, *42*,1830) discovered an important trace constituent of green-metallic odor reminiscent of geranium oil and carrot leaves. They termed this new odorant rose oxide and later attributed it the structure **1** (C.F. Seidel, D. Felix, A. Eschenmoser, K. Biemann, E. Palluy, M. Stoll, *Helv. Chim. Acta* **1961**, *44*, 598). Though in substance the very powerful, green-metallic odor of rose oxide is rather unpleasant, today it is extensively used in many fragrances to provide diffusivity, lift and naturalness, especially in the top note of perfumes.

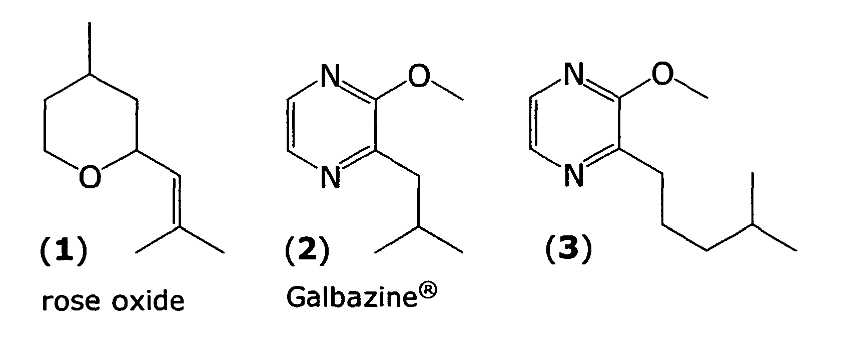

The methoxy pyrazines **2** and **3** represent another class of very powerful green odorants. **2** was first discovered by R. G. Buttery et al. in green bell pepper oil (R. G. Buttery, R. M. Seifert, R. E. Lundin, D. G. Guadagni, L C. Ling, *Chem. Ind.* **1969**, 490) and later also found in galbanum oil (A. F. Bramwell, J. W. K. Burrell, G. Riezebos, *Tetrahedron Lett.***1969***,* 3215). It is reminiscent of pepperoni, galbanum oil and green peas, and has been introduced into perfumery successfully as Galbazine^{®}. Isohexyl methoxy pyrazine (**3**) with its more pleasant green, vegetable and hyacinth odor is also frequently used in perfumery, though in very low concentration because of its exceptional strength. Yet, even in high dilutions, all these pyrazines have harsh, unpleasant by-odors, which limit their use in perfumery.

Unfortunately, blending of the aforementioned materials has not led to any interesting odor tonalities without harsh, unpleasant or metallic-bumt aspects being noticeable.

Accordingly, there is a need to provide new ingredients to develop interesting odor tonalities that have proven inaccessible by simple blending of these known compounds.

Surprisingly, it has now been found that certain 3,6-dihydro-2H-pyrans possess the characteristic odor notes of methoxy pyrazines, but do not exhibit the harsh by-notes that prohibit the extensive use of these pyrazines in perfumery. Facets of rose oxides are also present in these compounds leading to overall interesting, novel odor tonalities.

Therefore, the invention provides in a first aspect a compound of the general formula (I), 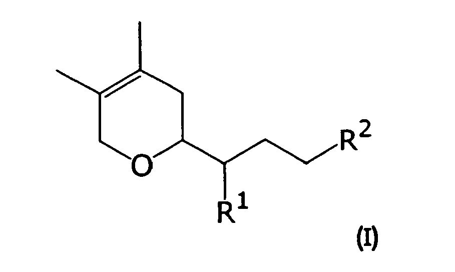 wherein R¹ = H, CH₃ or CH₂CH₃,
and R² = H, CH₃, CH₂CH₃ or CH₂CH₂CH₃, provided that when R¹ is H, R² is H.

The compounds of formula (I) may comprise more than one chiral centre and as such they may exist as a mixture of enantiomers and diastereomers, or they may be resolved as enantiomerically and diastereomerically pure forms. However, resolving stereoisomers adds to the complexity of manufacture and purification of these compounds and so it is preferred to use a compound of formula (I) as a mixture of its stereoisomers simply for economic reasons. However, if it is desired to prepare pure stereoisomers, this may be achieved according to methodology known in the art.

Compounds of formula (I) may be prepared according to techniques known in the art using commercially-available starting materials, or materials that can be easily prepared from known starting materials. The compounds may be prepared according to a hetero-Diels-Alder reaction using 2,3-dimethyl-1,3-butadiene and an appropriately substituted aldehyde, for example butyraldehyde may be employed in the formation of compound (7) in Table 1. Reaction conditions for these Diels-Alder reactions are more fully described in the Examples set forth below. The skilled person will appreciate that other compounds of the present invention may be prepared using an appropriately substituted aldehyde.

In another aspect of the present invention there is provided the use of a compound of the general formula (I) or mixtures thereof as a fragrance ingredient wherein,
R¹ = H, CH₃ or CH₂CH₃, and
R² = H, CH₃, CH₂CH₃ or CH₂CH₂CH₃.

Compounds described hereinabove possess unique, character-donating aspects that makes them suitable for a broad range of possible applications in perfumery. Furthermore, because the compounds do not possess the harsh unpleasant by-notes of the pyrazines exemplified by (2) and (3) above, they may confer a fresh green touch to virtually all kinds of perfume compositions, in virtually all kinds of applications from fine fragrances to cosmetics and detergents, e.g. in shampoos, skin-care products or soaps. Depending on the dosage, the effects can extend from providing more naturalness and character to building up hyacinth or petigrain or other green accords. The use is neither limited to any type of perfume nor to any special olfactory directions, odorants or classes of substances. A few illustrative examples of classes of substances, which harmonise equally well are:
- ethereal oils and extracts, e.g. angelica oil, basilic oil, bergamot oil, carrot seed oil, citrus oils, coriander oil, galbanum oil, geranium oil, jasmin absolute, neroli oil, orris oil, petitgrain oil, rose oil, or violet leaf oil;
- alcohols, e.g. citronellol, cinnamic alcohol, dihydromyrcenol, Ebanol^{®}, Florol^{®}, geraniol, linalool, Mayol^{®}, phenylethyl alcohol or terpineol ;
- aldehydes and ketones, e.g. decanal, *alpha*-hexylcinnamaldehyd, hydroxy-citronellal, isoeugenol, Iso E Super^{®}, Isoraldeine^{®}, Hedione^{®}, methylionone or vanillin;
- ether and acetals, e.g. Ambrox^{®}, 2-(1-ethoxyethoxy)ethyl benzene, geranyl methyl ether, Magnolan^{®}, 2-(1-propoxyethoxy)ethyl benzene, or rose oxide (1).
- esters and lactones, e.g. benzyl acetate, benzyl salicylate, coumarin, γ-decalactone, ethylene brassylate, Thibetolide^{®}, γ-undecalactone.
- heterocycles, e.g. indol, Galbazine^{®} (2) or 6-/8-isobutylchinoline.

Compounds of formula (I) that are particularly preferred for their fragrance properties are set forth in Table 1.

**Table 1. Compounds of general formula (I) having R¹ and R² as indicated**

| Compound | R¹ | R² |
|---|---|---|
| 4 | H | Methyl |
| 5 | H | Ethyl |
| 6 | H | Propyl |
| 7 | H | H |
| 8 | Ethyl | H |
| 9 | Methyl | H |
| 10 | Methyl | Methyl |

Having regard to compounds in Table 1, compound 4, compound 5 and compound 6 are known compounds, however, the olfactory properties of these compounds have not been previously disclosed.

Compounds according to the formula (I) may be employed in any of the fragrance compositions referred to above in widely varying amounts having regard to the other fragrance ingredients employed and depending on the fragrance accord that a perfumer is trying to achieve. Generally however, one may employ about 0.01 to 1.0% by weight in fine fragrances and about 0.01 to 10% by weight in other perfumed products.

In addition to their admixture with other fragrance ingredients, the compounds of formula (I) may be admixed with one or more excipients conventionally used in conjunction with fragrances in fragrance compositions, for example carrier materials, and other auxiliary agents commonly used in the art such as solvents, preservatives, colourants and the like. The invention therefore also provides
- a fragrance composition comprising at least one compound as hereinabove defined; and
- a method of conferring a desired fragrance on a fragrance composition, comprising the blending with known fragrance ingredients and/or excipients of at least one compound as hereinabove defined.

The invention is now illustrated with reference to the following nonlimiting examples.

### Examples

### 4,5-Dimethyl-2-propyl-3,6-dihydro-2H-pyran (Compound 7):

2-Nitropropane (1.8 ml, 20 mmol) was added at 0 °C to a stirred suspension of aluminium trichloride (2.67 g, 20 mmol) in dichloromethane (50 ml). A solution of butanal (14.4 g, 200 mmol) in dichloromethane (30 ml) was added, followed by dropwise addition of 2,3-dimethyl-1,3-butadiene (24.6 g, 300 mmol) at 0 °C. After stirring for 3h at this temperature, and for 1h at ambient temperature, the reaction mixture was poured into water (400 ml) and the product extracted with pentane (3x 300 ml). The combined organic extracts were dried, concentrated in vacuo, and purified by silica-gel FC (pentane/MTBE, 99:1) to provide the title compound **7** (3.55 g, 12%).

Odor: green, spicy, rose oxide, vegetable. - IR (neat): ν =1098 cm⁻¹ (ν C-O-C), 1386 (δ CH₃), 1449 (δ CH₂). -¹H-NMR (CDCl₃): δ = 0.93 (t, *J*= 7.0 Hz, 3H, 5'-H₃), 1.34-1.57 (m, 4H, 1'-H₂-2'-H₂), 1.53 / 1.63 (2s, 6H, 4-,5-Me), 1.80 (d, *J*= 16.5 Hz, 1H, 3-H_{b}), 1.93 (br. t, *J*= 16.5 Hz, 1H, 3-Hₐ), 3.47 (m_{c}, 1H, 2-H), 3.93 /4.01 (2d, *J*= 15.5 Hz, 2H, 6-H₂). - ¹³C-NMR (CDCl₃): δ = 13.8 (q, 5-Me),14.1 (q, C-3'), 18.3 (q, 4-Me),18.6 (C-2'), 36.7 (t, C-3), 38.0 (t, C-1'), 69.8 (t, C-6), 73.9 (d, C-2), 123.5 / 124.3 (2s, C-4,-5). - MS (EI): *m*/*z* (%) = 41 (45) [C₃H₅⁺], 55 (55) [C₄H₇⁺], 67 (100) [C₅H₇⁺], 82 (99) [C₆H₁₀⁺], 111 (19) [M⁺-C₃H₇], 139 (21) [M⁺-CH₃], 154 (67) [M⁺].

### 2-(1-Ethylpropyl)-4,5-dimethyl-3,6-dihydro-2H-pyran (Compound 8):

2-Nitropropane (1.8 ml, 20 mmol) was added at -10 °C to a stirred suspension of aluminium trichloride (2.67 g, 20 mmol) in dichloromethane (80 ml). At this temperature, 2-ethylbutanal (20.0 g, 200 mmol) was added dropwise during 20 min, followed by 2,3-dimethyl-1,3-butadiene (24.6 g, 300 mmol) during 30 min. After further stirring at this temperature for 30 min, and for 1h at ambient temperature, the reaction mixture was poured into water (300 ml) and the product extracted with MTBE (3x 300 ml). The combined organic extracts were dried, concentrated in vacuo, and purified by silica-gel FC (pentane/MTBE, 100:1, *R*_{f}= 0.47) to provide the title compound **8** (5.53 g, 15%).

Odor: green, carrot leaves, rose oxide, metallic, green peas, galbanum. - IR (neat): ν = 1099 cm⁻¹ (v C-O-C), 1384 (δ CH₃), 1461 (δ CH₂). - ¹H-NMR (CDCl₃): δ = 0.88 (t, *J*= 7.5 Hz, 6H, 3'-,2"'-H₂),1.53 /1.64 (2s, 6H, 4-,5-Me), 1.19-1.50 (m, 5H, 1'-H, 2'-,1"'-H₂), 1.74 (br. d, *J*= 16.0 Hz, 1H, 3-H_{b}), 2.03 (br. t, *J* = 16.0, 1H, 3-Hₐ), 3.41 (m_{c}, 1H, 2-H), 3.93 / 3.99 (2d, *J*=15.5 Hz, 2H, 6-H₂).-¹³C-NMR (CDCl₃): δ = 11.2 / 11.3 (2q, C-3',-2"), 13.7 (q, 5-Me), 18.4 (q, 4-Me), 21.2 / 21.3 (2t, C-2',-1"'), 33.5 (t, C-3), 45.5 (d, C-1'), 70.1 (t, C-6), 75.8 (d, C-2),123.8/124.4 (2s, C-4,-5). - MS (EI): *m*/*z* (%) = 41 (45) [C₃H₅⁺], 55 (54) [C₄H₇⁺], 67 (66) [C₅H₇⁺], 83 (100) [C₆H₁₁⁺],111 (65) [M⁺-C₅H₁₁],182 (28) [M⁺].

### 2-Butyl-4,5-dimethyl-3,6-dihydro-2H-pyran (Compound 4):

2-Nitropropane (1.8 ml, 20 mmol) was added at 0 °C to a stirred suspension of aluminium trichloride (2.67 g, 20 mmol) in dichloromethane (80 ml). Pentanal (17.2 g, 200 mmol) was added at -78 °C, followed by 2,3-dimethyl-1,3-butadiene (24.6 g, 300 mmol). The cooling bath was removed and the reaction was stirred at room temperature for 3h. The mixture was then poured into water (400 ml), and the product extracted with pentane (3x 400 ml). The organic extracts were combined and dried, the solvent was evaporated in vacuo, and the resulting residue purified by distillation to furnish at 34-38 °C / 0.05 mbar the title compound **4** (7.87 g, 12%).

Odor: green, spicy, rose oxide, hyacinth. - IR (neat): ν = 1100 cm⁻¹ (ν C-O-C), 1387 (δ CH₃), 1450 (δ CH₂). -¹H-NMR (CDCl₃): δ = 0.91 (t, *J*= 7.0 Hz, 3H, 5'-H₃), 1.30-1.58 (m, 6H, 1'-H₂-3'-H₂), 1.53 / 1.63 (2s, 6H, 4-,5-Me), 1.80 (d, *J*= 16.0 Hz, 1H, 3-H_{b}), 1.94 (br. t, *J*= 16.0 Hz, 1H, 3-Hₐ), 3.45 (m_{c}, 1H, 2-H), 3.93/4.01 (2d, *J*= 15.5 Hz, 2H, 6-H₂). -¹³C-NMR (CDCl₃): δ = 13.8 (q, 5-Me), 14.0 (q, C-4'), 18.3 (q, 4-Me), 22.7 (t, C-3'), 27.7 (t, C-2'), 35.6 / 36.7 (t, C-3,-1'), 69.8 (t, C-6), 74.2 (d, C-2), 123.5 / 124.3 (2s, C-4,-5). - MS (EI): *m*/*z* (%) = 41 (36) [C₃H₅⁺], 55 (41) [C₄H₇⁺], 67 (79) [C₅H₇⁺], 82 (100) [C₆H₁₀⁺], 85 (58) [C₅H₁₀⁺], 111 (20) [M⁺-C₄H₉], 153 (16) [M⁺-CH₃], 168 (54) [M⁺].

### 4,5-Dimethyl-2-pentyl-3,6-dihydro-2H-pyran (Compound 5):

2-Nitropropane (1.8 ml, 20 mmol) was added at -10 °C to a stirred suspension of aluminium trichloride (2.67 g, 20 mmol) in dichloromethane (80 ml). At this temperature, a solution of hexanal (20.0 g, 200 mmol) in dichloromethane (80 ml) was added within 15 min, followed by dropwise addition of 2,3-dimethyl-1,3-butadiene (24.6 g, 300 mmol) in a period of another 15 min. After further stirring at 0 °C for 90 min, the cooling bath was removed, and another portion of 2,3-dimethyl-1,3-butadiene (24.6 g, 300 mmol) was added dropwise with stirring at room. Stirring was continued at ambient temperature for 14 h, prior to pouring the reaction mixture into water (400 ml) and extraction of the product with MTBE (3x 400 ml). The combined organic extracts were dried, the solvent was removed under reduced pressure, and the resulting residue purified by distillation in vacuo to provide at 53-58 °C / 0.30-0.35 mbar the title compound **5** (5.41 g, 15%).

Odor: Strong, green peas, vegetable, isohexyl methoxy pyrazine, lily and hyacinth. - IR (neat): ν = 1102 cm⁻¹ (ν C-O-C), 1386 (δ CH₃), 1449 (δ CH₂), 1691 (ν C=C).⁻¹H-NMR (CDCl₃): δ = 0.89 (t, *J=* 7.0 Hz, 3H, 5'-H₃), 1.27-1.69 (m, 8H, 1'-H₂-4'-H₂), 1.53 / 1.59 (2s, 6H, 4-,5-Me), 1.80 (d, *J=* 16.0 Hz, 1H, 3-H_{b}), 1.94 (br. t, *J=* 16.o Hz, 1H, 3-Hₐ), 3.45 (m_{c}, 1H, 2-H), 3.93/4.01 (2d, *J=* 15.5 Hz, 2H, 6-H₂).-¹³C-NMR (CDCl₃)_{:} δ = 13.8 (q, 5-Me), 14.0 (q, C-5'), 18.3 (q, 4-Me), 22.6 (t, C-4'), 25.1 (t, C-2'), 31.9 (t, C-3'), 35.8 / 36.7 (t, C-3,-1'), 69.8 (t, C-6), 74.2 (d, C-2), 123.5 / 124.3 (2s, C-4,-5). - MS (EI): *m*/*z* (%) = 41 (32) [C₃H₅⁺], 55 (38) [C₄H₇⁺], 67 (64) [C₅H₇⁺], 82 (100) [C₆H₁₀⁺], 111 (20) [M⁺-C₅H₁₁], 167 (11) [M⁺-CH₃],182 (37) [M⁺].

### 2-Hexyl-4,5-dimethyl-3,6-dihydro-2H-pyran (Compound 6):

2-Nitropropane (1.8 ml, 20 mmol) was added at -10 °C to a stirred suspension of aluminium trichloride (2.67 g, 20 mmol) in dichloromethane (80 ml). Heptanal (22.8 g, 200 mmol) was added dropwise at this temperature during 15 min, followed by dropwise addition of 2,3-dimethyl-1,3-butadiene (24.6 g, 300 mmol) during 20 min. The reaction was stirred -10°C for 15 min., and at room temperature for 1h. Another portion of 2,3-dimethyl-1,3-butadiene (24.6 g, 300 mmol) was added during 20 min, and stirring was continued at room temperature for 15 h. The mixture was then poured into water (300 ml), and the product extracted with MTBE (3x 300 ml). The organic extracts were combined and dried, the solvent was evaporated in vacuo, and the resulting residue purified by FC (pentane/ether, 100:1) on silica gel to provide the title compound **6** (2.78 g, 7%).

Odor: Strong, green peas, parsley, vegetable, pyrazine, roots. - IR (neat): ν = 1104 cm⁻¹ (ν C-O-C), 1456 (δ CH₂), 1386 (δ CH₃), 1693 (v C=C).-¹H-NMR (CDCl₃): δ = 0.88 (t, *J*= 7.0 Hz, 3H, 6'-H₃),1.29-1.69 (m, 10H, 1'-H₂-5'-H₂), 1.53/1.63 (2s, 6H, 4-,5-Me), 1.80 (d, *J*= 16.0 Hz, 1H, 3-H_{b}), 1.94 (br. t, *J*= 16.0 Hz, 1H, 3-Hₐ), 3.45 (m_{c}, 1H, 2-H), 3.93 / 4.01 (2d, *J*= 16.0 Hz, 2H, 6-H₂).-¹³C-NMR (CDCl₃): δ = 13.8 (q, 5-Me), 14.1 (q, C-6'), 18.3 (q, 4-Me), 22.6 (t, C-5'), 25.4 (t, C-2'), 29.4 (t, C-3'), 31.8 (t, C-4'), 35.9 / 36.7 (t, C-3,-1'), 69.8 (t, C-6), 74.3 (d, C-2),123.5 / 124.3 (2s, C-4,-5).- MS (EI): *m*/*z* (%) = 41 (24) [C₃H₅⁺], 55 (29) [C₄H₇⁺], 67 (51) [C₅H₇⁺], 82 (100) [C₆H₁₀⁺], 85 (52) [C₆H₁₃⁺], 111 (21) [M⁺-C₆H₁₃],181 (13) [M⁺-CH₃],196 (37) [M⁺].

### 4,5-Dimethyl-2-(1-methylpropyl)-3,6-dihydro-2H-pyran (Compound 9):

To a suspension of aluminium trichloride (2.67 g, 20 mmol) in dichloromethane (50 ml) was added 2-nitropropane (1.8 ml, 20 mmol) at 0 °C At the same temperature, a solution of 2-methylbutanal (17.2 g, 200 mmol) and 2,3-dimethylbutadiene (24.6 g, 300 mmol) in dichloromethane (30 ml) was added dropwise with stirring, and stirring was continued at 0 °C for 30 min. and at room temperature for 2 h, prior to the addition of another portion of 2,3-dimethylbutadiene (24.6 g, 300 mmol). After further stirring at room temperature for 15 h, the reaction mixture was poured into water (300 ml) and the product was extracted with MTBE (3x 300 ml). The combined organic extracts were dried and concentrated under reduced pressure. The resulting crude product was purified by distillation to provide at 90-92 °C / 20 mbar the title compound **9** (5.76 g, 17%).

Odor: Rose oxide, green, aromatic. - IR (neat): ν =1099 cm⁻¹ (ν C-O-C), 1383 (δ CH₃), 1457 (δ CH₂). -¹H-NMR (CDCl₃): δ = 0.91 / 0.97 (2d, *J*= 7.0 Hz, 3H, 1'-Me), 0.92 / 0.93 (2t, *J*= 7.0 Hz, 3H, 4'-H₃), 1.15-1.64 (m, 3H, 1'-H,2'-H₂), 1.56/1.67 (2s, 6H, 4-,5-Me), 1.77 (2 br. d, *J*= 16.0 Hz, 1H, 3-H_{b}), 2.05 / 2.08 (2 br. t, *J*= 16.0 Hz, 1H, 3-Hₐ), 3.30 (m_{c}, 1H, 2-H), 3.97 / 4.03 (2d, *J*= 15.5 Hz, 2H, 6-H₂). -¹³C-NMR (CDCl₃): δ = 11.3 /11.5 (q, C-3'), 13.7 / 13.8 / 14.2 / 14.6 (4q, 5-Me, C-4'), 18.4 /18.5 (2q, 4-Me), 25.0 / 25.4 (2t, C-2'), 33.1 / 33.7 (2t, C-3), 39.2 / 39.3 (2d, C-1'), 70.1 /70.2 (2t, C-6), 77.9 / 77.9 (2d, C-2), 123.7 / 123.8 / 124.4 / 124.4 (4s, C-4,-5). - MS (EI): *m*/*z* (%) = 41 (44) [C₃H₅⁺], 55 (59) [C₄H₇⁺], 67 (75) [C₅H₇⁺], 83 (100) [C₆H₁₁⁺], 111 (58) [M⁺-C₄H₉], 153 (2) [M⁺-CH₃], 168 (45) [M⁺].

### 4,5-Dimethyl-2-(1-methylbutyl)-3,6-dihydro-2H-pyran (Compound 10):

2-Nitropropane (1.8 ml, 20 mmol) was added at -10 °C to a stirred suspension of aluminium trichloride (2.67 g, 20 mmol) in dichloromethane (80 ml). At this temperature, 2-methylpentanal (20.0 g, 200 mmol) was added dropwise during 20 min, followed by 2,3-dimethyl-1,3-butadiene (24.6 g, 300 mmol) during 30 min. After further stirring at this temperature for 30 min, and for 1h at ambient temperature, the reaction mixture was poured into water (300 ml) and the product extracted with MTBE (3x 300 ml). The combined organic extracts were dried, concentrated in vacuo, and purified by silica-gel FC (pentane/MTBE, 100:1, *R*_{f}= 0.21) to provide the title compound **10** (7.43 g, 20%).

Odor: Green, vegetable, aromatic, tea-like. - IR (neat): ν = 1101 cm⁻¹ (ν C-O-C), 1383 (δ CH₃), 1456 (δ CH₂). -¹H-NMR (CDCl₃): δ = 0.88 / 0.94 (2d, *J* = 7.0 Hz, 3H, 1'-Me), 0.89 / 0.90 (2t, *J*= 7.0 Hz, 3H, 4'-H₃), 1.10-1.61 (m, 5H, 1'-H, 2'-,3'-H₂'), 1.52 / 1.64 (2s, 6H, 4-,5-Me), 1.73 (br. d, *J=* 16.5 Hz, 1H, 3-H_{b}), 2.05 (m_{c}, 1H, 3-Hₐ), 3.26 (m_{c}, 1H, 2-H), 3.94 / 4.00 (2 br. d, J=15.5 Hz, 2H, 6-H₂). - ¹³C-NAAR (CDCl₃): δ = 13.8 / 14.3 / 14.7 / 15.1 (4q, 5-Me, C-4'), 18.3 / 18.4 (2q, 4-Me), 20.1 / 20.2 (2t, C-2'), 33.0 / 33.6 (2t, C-3'), 34.6 / 35.0 (2t, C-3), 37.3 / 37.5 (2d, C-1'), 70.1 / 70.2 (2t, C-6), 78.1 / 78.2 (2d, C-2),123.6 / 123.7 / 124.4 / 124.4 (4s, C-4,-5). - MS (EI): *m*/*z (%)* = 43 (44) [C₃H₇⁺], 55 (55) [C₄H₇⁺], 67 (67) [C₅H₇⁺], 83 (100) [C₆H₁₁⁺], 111 (63) [M⁺-C₅H₁₁], 167 (2) [M⁺-CH₃], 182 (30) [M⁺].

### Floral-green perfume oil for fabric softeners:

| | compound/ingredient | parts by weight 1/1150 |
|---|---|---|
| 1. | Agrumex (*ortho-tert* Butylcyclohexyl acetate) | 5 |
| 2. | Aubepine (*para*-Methoxybenzaldehyde) / *para*-cresol | 15 |
| 3. | Benzyl acetate | 25 |
| 4. | Benzyl salicylate | 35 |
| 5. | *para-tert* Butylcyclohexyl acetate | 106 |
| 6. | Butyl hydroxy toluene | 2 |
| 7. | Cinnamic alcohol | 5 |
| 8. | Citronellol, extra quality | 50 |
| 9. | Coumarine, pure, crystalline | 10 |
| 10. | Damascenone @ 1% in DPG | 10 |
| 11. | *alpha*-Damascone @ 10% in DPG | 4 |
| 12. | *gamma*-Decalactone | 4 |
| 13. | Decanal | 4 |
| 14. | Dihydromyrcenol | 40 |
| 15. | DPG (Dipropylene glycol) | 20 |
| 16. | Ebanol (3-Methyl-5-(2,2,3-trimethyl-3-cyclopenten-1-yl)-4-penten-2-ol) | 3 |
| 17. | Ethyl vanillin | 3 |
| 18. | Eugenol, pure @ 10°/ in DPG | 10 |
| 19. | Fixolide (6-Acetyl-1,1,2,4,4,7-hexamethyltetralin) | 10 |
| 20. | Galaxolide 50 PHT (4,6,6,7,8,8-Hexamethyl-1,3,4,6,7,8-hexahydrocyclopenta[g]benzopyran) | 100 |
| 21. | Gardenol (1-Phenylethyl acetate) | 15 |
| 22. | Geraniol | 40 |
| 23. | *alpha*-Hexylcinnamaldehyde | 90 |
| 24. | 6-/8-Isobutylquinoline @ 10% in DPG | 5 |
| 25. | Isoraldeine 70 (8-Methylionone) | 50 |
| 26. | Lilial (2-Methyl-3-(4-*tert*-butylphenyl)propanal) | 80 |
| 27. | Linalool | 80 |
| 28. | Manzanate (Ethyl 2-methyl pentanoate) @ 10% in DPG | 5 |
| 29. | Mayol (para-Isopropylcyclohexylmethanol) | 2 |
| 30. | Menthone @ 10% in DPG | 5 |
| 31. | 2-Methyldecanal @ 10% in DPG | 3 |
| 32. | 6-Methylhept-5-en-2-one @ 10% in DPG | 10 |
| 33. | Methyl isoeugenol | 5 |
| 34. | Methyl salicylate @ 10% in DPG | 5 |
| 35. | Nerol, extra quality | 10 |
| 36. | Okoumal (2,4-Dimethyl-2-(1,1,4,4-tetramethyl-tetralin-6-yl)-1,3-dioxolane) | 15 |
| 37. | 2-Phenylethanol | 25 |
| 38. | Strawberry pure (Ethyl 3-methyl-3-phenyl-glycidate) @ 10 % in DPG | 10 |
| 39. | Terpenyl acetate | 40 |
| 40. | Terpineol, pure | 70 |
| 41. | *gamma*-Undecalactone | 4 |
| 42. | Undecanal | 40 |
| 43. | 10-Undecenal @ 10% in DPG | 20 |
| 44. | Verdyl acetate | 30 |
| 45. | Veloutone (2,2,5-Trimethyl-5-pentylcyclopentanone) @ 10 % in DPG | 10 |
| 46. | Ylang ylang oil | 15 |
| 47. | Compound 5 @ 10% in DPG | 5 |
| | | 1150 |

Compound 5 conveys to this perfume freshness and lift, and in particular petigrain-type aspects. It combines very well with the aldehydes, and turns the top note of the fragrance oil more eau de cologne-like; thereby, **5** attenuates the functional aspects of the fragrance and the fabric softener becomes overall more sophisticated, more refined.

### Floral-marine fragrance for soap:

| | compound/ingredient | parts by weight 1/820 |
|---|---|---|
| 1. | Agrumex *(2-tert* Butylcyclohexyl acetate) | 30 |
| 2. | Ambrofix (3-Methyldodechydro-6,6-9a-oxidotetranorlabdane) @ 10% in DEP | 2 |
| 3. | Aubepine (*para*-Methoxybenzaldehyde) / *para*-cresol @ 10°/ in DPG | 2 |
| 4. | Bergamot Givco 104 | 80 |
| 5. | *iso-*Butyl benzoate | 30 |
| 6. | Cassis Base 345-F CS | 2 |
| 7. | Cetone V (1-(2,6,6-Trimethyl-2-cyclohexen-1-yl)-hepta-1,6-dien-3-one) | 2 |
| 8. | Citral | 5 |
| 9. | Citron Ess Reconst 1385 | 10 |
| 10. | Citronellyl nitrile | 1 |
| 11. | *beta*-Damascone @ 1% in DPG | 20 |
| 12. | 2,4-Dimethyl-3-cyclohexenecarboxaldehyde | 4 |
| 13. | DPG (Dipropylene glycol) | 83 |
| 14. | Floralozone (*alpha*,*alpha*-Dimethyl-4-ethylbenzenepropanal) | 5 |
| 15. | Galaxolide 50 PHT (4,6,6,7,8,8-Hexamethyl-1,3,4,6,7,8-hexahydrocyclopenta[g]benzopyran) | 30 |
| 16. | Geraniol, extra quality | 5 |
| 17. | Hedione | 100 |
| 18. | *cis*-3-Hexenol @ 10% in DPG | 5 |
| 19. | *cis*-3-Hexenyl acetate @ 10% in DPG | 5 |
| 20. | *cis*-3-Hexenyl butyrate @ 10% in DPG | 5 |
| 21. | *cis*-3-Hexenyl hexenoate @ 10% in triethyl citrate | 10 |
| 22. | *alpha*-Hexylcinnamaldehyde | 100 |
| 23. | *beta*-Ionone | 15 |
| 24. | *cis*-Jasmone | 2 |
| 25. | Linalool | 50 |
| 26. | Mandarinal Base (Firmenich) | 3 |
| 27. | Methyl 2-nonynoate @ 1% in DPG | 5 |
| 28. | Nerolidol | 100 |
| 29. | Neroli oil | 2 |
| 30. | Nutmeg (mace) oil @ 10 % in DPG | 2 |
| 31. | Phenylethyl phenylacetate | 3 |
| 32. | Terpenyl Acetate | 50 |
| 33. | Terpineol, pure | 20 |
| 34. | Terpinolene | 10 |
| 35. | *gamma* Undecalactone | 2 |
| 36. | Compound 5 @ 10% in DPG | 20 |
| | | 820 |

Compound 5 harmoniously combines the hesperidic, floral-marine and green notes of the fragrance; thereby, it rounds off the composition and increases its radiance. In addition, it introduces naturalness and increases the character of that fragrance. At this relatively high dosage, 5 additionally brings in facets of petitgrain oil and jasmone; overall, 5 conveys to the fragrance its unique texture, its fresh and hygienic but also soft and caressing character.

### Essai for a male fine fragrance:

| | compound/ingredient | parts by weight 1/1000 |
|---|---|---|
| 1. | Ambrein, pure Base (Biolande) | 2 |
| 2. | Anise oil | 2 |
| 3. | Artemisia (Armoise) oil | 3 |
| 4. | Bergamotte Givco 104 | 80 |
| 5. | Benzyl salicylate | 50 |
| 6. | Birch Leaf Givco 166 | 4 |
| 7. | Bornyl acetate | 5 |
| 8. | Coumarine, pure | 5 |
| 9. | Cyclal C (2,4-Dimethyl-3-cyclohexene carboxaldehyde) | 1 |
| 10. | *delta*-Damascone | 1 |
| 11. | Dihydromyrcenol | 120 |
| 12. | DPG (Dipropylene glycol) | 95 |
| 13. | Evernyl (Methyl 3,6-dimethylresorcylate) | 8 |
| 14. | Fir balsam resinoide | 2 |
| 15. | Galaxolide 50 PHT (4,6,6,7,8,8-Hexamethyl-1,3,4,6,7,8-hexahydrocyclopenta[g]benzopyran) | 180 |
| 16. | Galbanone 10 (1-(5,5-Dimethyl-1-cyclohexen-1-yl)-4-penten-1-one) | 5 |
| 16. | Gardenol (1-Phenylethyl acetate) | 2 |
| 17. | Geranium oil | 8 |
| 18. | Hedione | 150 |
| 19. | Iso E Super | 160 |
| 20. | Kephalis (4-(1-Ethoxyethenyl)-3,3,5,5-tetramethylcyclohexanone) | 15 |
| 21. | Lavandin Grosso oil | 3 |
| 22. | Linalool | 40 |
| 23. | Melonal (2,6-Dimethylhept-5-enal) @ 10% in DPG | 1 |
| 24. | Nutmeg (mace) oil | 5 |
| 25. | Patchouli oil (iron free) | 8 |
| 26. | Radjanol | 40 |
| 36. | Compound 5 | 5 |
| | | 1000 |

Compound 5 conveys to this male fine fragrance essai its fresh facet: a green and invigorating botanical freshness, clean, clear and compelling. It enriches the classical top of this modem fougere by new aspects, increases radiance and diffusion, and harmonises and stages the spicy accord of anise, artemisia and nutmeg oils. In result, this masculine scent acquires a herbal crispness that does not disturb the mysterious sensuality of the woody-musky-mossy-coumarine fond, but instead accentuates it.

## Claims

1. A compound of the general formula (I) 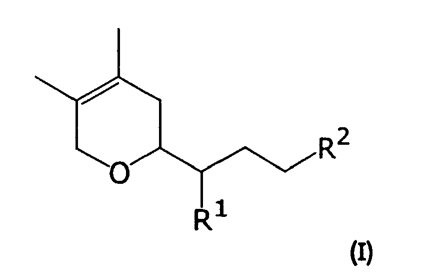 wherein,
R¹ = H, CH₃ or CH₂CH₃,
R² = H, CH₃, CH₂CH₃ or CH₂CH₂CH₃,
provided that when R¹ is H, R² is H.

2. A compound according to claim 1 selected from the group consisting of 2-(1-ethylpropyl)-4,5-dimethyl-3,6-dihydro-2H-pyran; 4,5-dimethyl-2-propyl-3,6-dihydro-2H-pyran; 4,5-dimethyl-2-(1-methylpropyl)-3,6-dihydro-2H-pyran; 4,5-dimethyl-2-(1-methylbutyl)-3,6-dihydro-2H-pyran; or mixtures thereof.

3. The use as a fragrance ingredient of a compound of the formula (I) 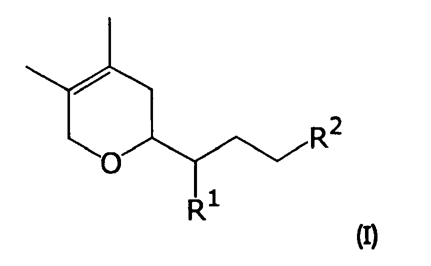 wherein,
R¹ is H, CH₃ or CH₂CH₃, and
R² is H, CH₃, CH₂CH₃, CH₂CH₂CH₃

4. Use according to claim 3 wherein the compound is selected from the group consisting of 2-butyl-4,5-dimethyl-3,6-dihydro-2H-pyran; 4,5-dimethyl-2-pentyl-3,6-dihydro-2H-pyran; 2-(1-ethylpropyl)-4,5-dimethyl-3,6-dihydro-2H-pyran; 2-hexyl-4,5-dimethyl-3,6-dihydro-2H-pyran; 4,5-dimethyl-2-propyl-3,6-dihydro-2H-pyran; 4,5-dimethyl-2-(1-methylpropyl)-3,6-dihydro-2H-pyran; 4,5-dimethyl-2-(1-methylbutyl)-3,6-dihydro-2H-pyran; or mixtures thereof.

5. A fragrance composition comprising at least one compound as defined in claim 3 or claim 4.

6. A method of conferring a desired fragrance on a fragrance composition, comprising the blending with known fragrance ingredients and/or excipients of at least one compound as defined in claim 3 or claim 4.

## Patentansprüche

1. Verbindung der allgemeinen Formel (I) 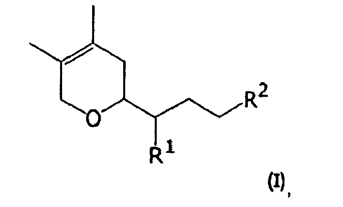 wobei
R¹ = H, CH₃ oder CH₂CH₃,
R² = H, CH₃, CH₂CH₃ oder CH₂CH₂CH₃,
mit der Maßgabe, dass R² für H steht, wenn R¹ für H steht.

2. Verbindung nach Anspruch 1, die ausgewählt ist aus der Gruppe, bestehend aus 2-(1-Ethylpropyl)-4,5-dimethyl-3,6-dihydro-2H-pyran, 4,5-Dimethyl-2-propyl-3,6-dihydro-2H-pyran, 4,5-Dimethyl-2-(1-methylpropyl)-3,6-dihydro-2H-pyran, 4,5-Dimethyl-2-(1-methylbutyl)-3,6-dihydro-2H-pyran, oder Gemische davon.

3. Verwendung einer Verbindung der Formel (I) 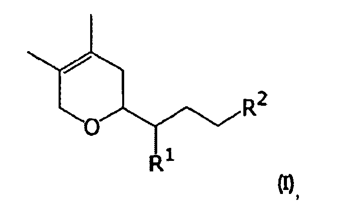 wobei
R¹ für H, CH₃ oder CH₂CH₃ steht und
R² für H, CH₃, CH₂CH₃ oder CH₂CH₂CH₃ steht, als Duftinhaltsstoff.

4. Verwendung nach Anspruch 3, wobei die Verbindung ausgewählt ist aus der Gruppe, bestehend aus 2-Butyl-4,5-dimethyl-3,6-dihydro-2H-pyran, 4,5-Dimethyl-2-pentyl-3,6-dihydro-2H-pyran, 2-(1-Ethylpropyl)-4,5-dimethyl-3,6-dihydro-2H-pyran, 2-Hexyl-4,5-dimethyl-3,6-dihydro-2H-pyran, 4,5-Dimethyl-2-propyl-3,6-dihydro-2H-pyran, 4,5-Dimethyl-2-(1-methylpropyl)-3,6-dihydro-2H-pyran, 4,5-Dimethyl-2-(1-methylbutyl)-3,6-dihydro-2H-pyran oder Gemischen davon.

5. Duftzusammensetzung, umfassend mindestens eine Verbindung, wie sie in Anspruch 3 oder Anspruch 4 definiert ist.

6. Verfahren eines Übertragens eines gewünschten Dufts auf eine Duftzusammensetzung, umfassend das Mischen mindestens einer Verbindung, wie sie in Anspruch 3 oder Anspruch 4 definiert ist, mit bekannten Duftinhaltsstoffen und/oder Hilfsstoffen.

## Revendications

1. Composé de formule générale (I) 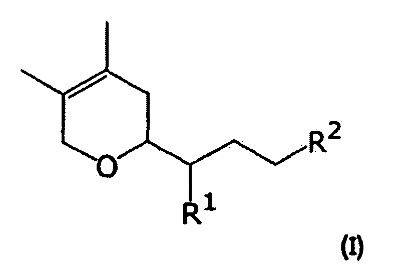 dans laquelle
R¹ = H, CH₃ ou CH₂CH₃,
R² = H, CH₃, CH₂CH₃ ou CH₂CH₂CH₃,
pourvu que lorsque R¹ représente H, R² représente H.

2. Composé selon la revendication 1, choisi dans le groupe constitué par le 2-(1-éthylpropyl)-4,5-diméthyl-3,6-dihydro-2H-pyranne; le 4,5-diméthyl-2-propyl-3,6-dihydro-2H-pyranne; le 4,5-diméthyl-2-(1-méthylpropyl)-3,6-dihydro-2H-pyranne; le 4,5-diméthyl-2-(1-méthylbutyl)-3,6-dihydro-2H-pyranne; ou leurs mélanges.

3. Utilisation comme ingrédient de parfum d'un composé de formule (I) 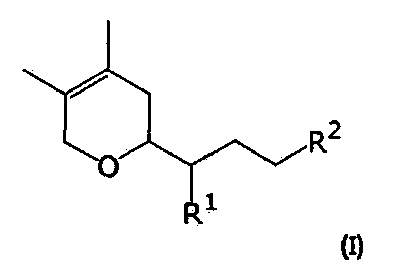 dans laquelle
R¹ représente H, CH₃ ou CH₂CH₃ et
R² représente H, CH₃, CH₂CH₃, CH₂CH₂CH₃.

4. Utilisation selon la revendication 3, dans laquelle le composé est choisi dans le groupe constitué par le 2-butyl-4,5-diméthyl-3,6-dihydro-2H-pyranne; le 4,5-diméthyl-2-pentyl-3,6-dihydro-2H-pyranne, le 2-(1-éthylpropyl)-4,5-diméthyl-3,6-dihydro-2H-pyranne; le 2-hexyl-4,5-diméthyl-3,6-dihydro-2H-pyranne; le 4,5-diméthyl-2-propyl-3,6-dihydro-2H-pyranne; le 4,5-diméthyl-2-(1-méthylpropyl)-3,6-dihydro-2H-pyranne; le 4,5-diméthyl-2-(1-méthylbutyl)-3,6-dihydro-2H-pyranne; ou leurs mélanges.

5. Composition de parfum comprenant au moins un composé tel que défini dans la revendication 3 ou la revendication 4.

6. Procédé pour conférer un parfum désiré à une composition de parfum, comprenant le mélange d'au moins un composé tel que défini dans la revendication 3 ou la revendication 4 avec des ingrédients de parfum connus et/ou des excipients.
